# EUROPEAN PATENT SPECIFICATION

(11) **EP 2 419 503 B1**
(45) Date of publication and mention of the grant of the patent: **19.12.2012**
(21) Application number: 10730692.0
(22) Date of filing: 12.04.2010
(51) Int. Cl.: C12M 3/04

(54) **METHOD OF PRODUCTION OF SYNCHRONIZED ADHERENTLY GROWING CELL LINES AND DEVICE FOR CARRYING OUT SAID METHOD**
VERFAHREN ZUR HERSTELLUNG SYNCHRONISIERTER ANHAFTEND WACHSENDER ZELLLINIEN UND VORRICHTUNG ZUR AUSFÜHRUNG DIESES VERFAHRENS
PROCÉDÉ DE PRODUCTION DE LIGNÉES CELLULAIRES EN CROISSANCE ADHÉRENTE SYNCHRONISÉE ET DISPOSITIF POUR LA MISE EN OEUVRE DUDIT PROCÉDÉ

(30) Priority: 16.04.2009 CZ 20090236; 16.04.2009 CZ 200921114 U
(43) Date of publication of application: 22.02.2012
(73) Proprietor: Univerzita Palackeho, 771 47 Olomouc (CZ)
(72) Inventor: MISTRIK, Martin, 779 00 Olomouc (CZ); BARTEK, Jiri, DK-2670 Greve (DK)
(74) Representative: Gabrielova, Marta
(86) International application number: PCT/CZ2010/000043
(87) International publication number: WO 2010/118709

(56) References cited:
- EP-A2- 1 284 285
- JP-A- 56 154 988
- US-A1- 2004 014 215

## Description

### Field of the Invention

The present invention relates to a new method of production of synchronized adherently growing cell lines that can be used especially in laboratories for basic and applied research, and it also describes construction of a device for carrying out said method.

### Background Art

Cell line growing techniques are an integral part of work for many biological, biotechnological and medical laboratories for basic and applied research. Cell lines are used for drug and chemical screening, for production of bio products and, last but not least, for the basic research and study of diseases including cancer, degenerative diseases, aging etc. One of the integral aspects of the cell population growth is the asynchronicity of the individual cells, which means that the cells go through the stages of the cell cycle at different pace and are thence forming a heterogeneous population of cells in various phases of the growth cycle. This heterogeneity of the cell population represents a serious problem for most experimental procedures because cells in different phases of the cell cycle have different physical, physiological, biochemical and genetic properties. Many experimental procedures therefore require only synchronized cell population as a model, in other words, only cells in a specific phase of the cell cycle. Achieving homogeneous synchronous cell population of a sufficient size is a complicated technological process. It can be achieved by either separation of the cells or by chemical synchronization of the cell growth. Both these methods are widely used depending on the laboratory instrumentation available, sometimes both methods are combined. The advantages and disadvantages of the methods most often used to achieve the synchronous cell population are summarized herein below.

Initiation methods are synchronization methods which use chemicals such as replication inhibitors or mitotic inhibitors, or change in the culture medium, such as serum deficiency, hormonal insufficiency or lack of growth factors, to initiate arrestation of cell proliferation in a certain phase of the cell cycle. Mechanistically, they usually employ a stress stimulus which activates pathways blocking further proliferation. The proliferation is usually arrested until the stress signal subsides by, e.g., washing out the inhibitor or suppling the serum, in some cases the proliferation inhibition is permanent.

Cell synchronization method on the basis of replication inhibitors uses mostly compounds such as hydroxyurea, thymidine and aphidicolin, as described, e.g., in Deborah S. Parris and Robert C. Bates: Synchronization of primary fetal cell cultures with hydroxyurea (1978, Methods in Cell Science 4, 745-747*),* Whitmore,G.F and Gulyas,S.: Synchronization of mammalian cells with tritiated thymidine (1966, Science 151, 691-694*)* or Fox, MH., et al.: Comparison of synchronized Chinese hamster ovary cells obtained by mitotic shake-off, hydroxyurea, aphidicolin, or methotrexate. (1987, Cytometry 8, 315-320*).* Hydroxyurea and thymidine cause the decrease of free deoxyribonucleotide triphosphates (dNTP), the main structural units of DNA, aphidicolin is a direct inhibitor of DNA polymerases. The inhibition of DNA replication synchronizes the cells on the transition between the G1 phase, i.e. the cell's preparation for replication, and S phase, i.e. the DNA replication. The advantage of this method is the possibility of reaching a very homogenous synchronized cell population by repeated exposition of the cells to the replication inhibitors and the fact that the replication inhibitors are usually cheap and available compounds. The disadvantage of this method is the stress of the exposition to the replication inhibitors which triggers a complex cell reaction with hardly predictable effects. These may interfere with the experimental procedure. Furthermore, the cells which are already in the replication process may suffer irreversible changes to their DNA because replication is generally a very delicate process and any interference causes a strong mutagenic and therefore irreversible effect. This is described, for instance, in Arnaudeau, C., et al.: Inhibition of DNA synthesis is a potent mechanism by which cytostatic drugs induce homologous recombination in mammalian cells (2000, Mutat. Res. 461, 221-228*),* Lundin, C., et al.: Different roles for nonhomologous end joining and homologous recombination following replication arrest in mammalian cells (2002, Mol. Cell Biol. 22, 5869-5878*),* Saintigny, Y., et al. : Characterization of homologous recombination induced by replication inhibition in mammalian cells (2001, EMBO J. 20, 3861-3870*),* Kurose,A., et al.: Effects of hydroxyurea and aphidicolin on phosphorylation of ataxia telangiectasia mutated on Ser 1981 and histone H2AX on Ser 139 in relation to cell cycle phase and induction of apoptosis (2006a, Cytometry A 69, 212-221*)* or Kurose,A., et al.: Synchronization in the cell cycle by inhibitors of DNA replication induces histone H2AX phosphorylation: an indication of DNA damage (2006b, Cell Prolif. 39, 231-240*).* Some lines after the application of the replication inhibitors immediately initiate apoptosis and therefore are absolutely unsuitable for this type of synchronization, as described, e.g., in Bolderson,E., et al.: ATM is required for the cellular response to thymidine induced replication fork stress (2004, Hum. Mol. Genet. 13, 2937-2945*),* Johnson, CA., et al.: Hydroxyurea induces apoptosis and regular DNA fragmentation in a Burkitt's lymphoma cell line. Biochim (1992, Biophys. Acta 1136, 1-4) or Woo, G.H., et al.: Molecular mechanisms of hydroxyurea(HU)-induced apoptosis in the mouse fetal brain (2006, Neurotoxicol. Teratol. 28, 125-134*).* Another considerable restriction is the time and labour intensity of this synchronization process, for example a common synchronization using thymidine blocks takes up to 100 hours for the normal human cell line and in addition to the simple cultivation also requires multiple thorough washing combined with 2-deoxycytidine application, as described in Clute, P. and Pines, J.: Temporal and spatial control of cyclin B1 destruction in metaphase (1999, Nat. Cell Biol. 1, 82-87*).*

Cell synchronization by serum deficiency forces the cells to cease their growth as a result of a stress stimulus generally referred to as starvation, which blocks start of DNA replication in the cell and stops the cell proliferation in the G1 phase, as described in articles Cooper,S.: Rethinking synchronization of mammalian cells for cell cycle analysis (2003, Cell Mol. Life Sci. 60, 1099-1106*),* or Wilmut, I., et al: Viable offspring derived from fetal and adult mammalian cells (1997, Nature 385, 810-813*).* The advantage of this method is the simplicity of the experimental procedure when the cells are not exposed to chemicals and therefore do not need complicated rinsing and there are no costs related to the purchase of chemicals and manipulation with them. The disadvantage is the fact that starvation is a stress causing a complex cell response with unpredictable effects. These may interfere with the planned experimental procedure. Many cell lines react to the starvation by initiating autophagocytosis, i.e. the process of obtaining missing compounds by degradation of some of the cell's own organelles, as described in Marie Stampe Ostenfeld: Anti-cancer agent siramesine is a lysosomotropic detergent that induces cytoprotective autophagosome accumulation. (2008, Autophagy*),* or by immediate initiation of apoptosis, see Lu, C., et al.: Serum starvation induces H2AX phosphorylation to regulate apoptosis via p38 MAPK pathway (2008, FEBS Lett. 582, 2703-2708*).* These lines are therefore completely unsuitable for this method.

Cell synchronization by mitotic inhibitor is a method combining initiation and separation. To initiate mitosis synchronization, chemicals interacting with tubulin are used. They cause defects in formation, breakdown and regulation of the mitotic spindle. Any problem concerning the mitotic spindle results in a cell stress and the completion of mitosis is blocked by activation of the so-called mitotic checkpoint. The cells arrested in mitosis are usually subsequently separated by using one of the separation techniques described below, mostly by the shaking or washing methods. The mitosis is most frequently blocked by microtubule poisons Colchicine, see Andreu, J.M. and Timasheff,S.N.: Tubulin bound to colchicine forms polymers different from microtubules (1982, Proc. Natl. Acad. Sci. U. S. A 79, 6753-6756*),* Andreu,J.M., et al.: Polymerization of the tubulin-colchicine complex: relation to microtubule assembly (1983 Biochemistry 22, 1556-1566*), Farrell, K. W. and* Wilson,L.: Proposed mechanism for colchicine poisoning of microtubules reassembled in vitro from Strongylocentrotus purpuratus sperm tail outer doublet tubulin (1980, Biochemistry 19, 3048-3054*),* Colcemide, see Stubblefield, E. et al.:, Synchronized mammalian cell cultures. I. Cell replication cycle and macromolecular synthesis following brief colcemid arrest of mitosis (1967, J Cell Physiol 69, 345-353*),* Nocodazole, see Lee,J.C., et al.: Effects of nocodazole on structures of calf brain tubulin (1980, Biochemistry 19, 6209-6215*)* or Zieve, G. W., et al: Production of large numbers of mitotic mammalian cells by use of the reversible microtubule inhibitor nocodazole. Nocodazole accumulated mitotic cells (1980, Exp. Cell Res. 126, 397-405*),* 2-methoxyestradiol described in EP 1 284 285 or in Atalla, H., et al.: 2-methoxyestradiol arrests cells in mitosis without depolymerizing tubulin (1996, Biochem. Biophys. Res. Commun. 228, 467-473*),* Attalla, H., et al.: Cytogenetic chromosomal preparations using 2-methoxyestradiol (1998, Cancer Genet. Cytogenet. 102, 139-141*),* or Rotenone, see Srivastava,P. and Panda,D.: Rotenone inhibits mammalian cell proliferation by inhibiting microtubule assembly through tubulin binding (2007, FEBS J 274, 4788-4801*).* The advantage of this method is that it is not technologically and economically demanding and if the separation step is added, the acquired cell fraction is very homogeneous with all the cells in the mitotic metaphase. The disadvantage of this method is that all mitotic inhibitors are highly toxic substances and their use affects not only the microtubuli of the mitotic spindle but also all other tubular structures of the cell, i.e., cytoskeleton and nucleoskeleton. The negative effects of the microtubule poisons are especially nuclear fragmentation, dispersal of Golgi apparatus and metabolic changes, as described in Minin, A.A.: Dispersal of Golgi apparatus in nocodazole-treated fibroblasts is a kinesin-driven process (1997, J Cell Sci. 110 (Pt 19), 2495-2505*).* Microtubule poisons are proved to be mutagens initiating irreversible genetic changes and a frequent cell response to their application is apoptosis and/or cessation of further cell proliferation, as described in Davoodpour,P. and Landstrom,M.: 2-Methoxyestradiol-induced apoptosis in prostate cancer cells requires Smad7 (2005, J Biol. Chem. 280, 14773-14779*),* Fox,MH., et al.: Comparison of synchronized Chinese hamster ovary cells obtained by mitotic shake-off, hydroxyurea, aphidicolin, or methotrexate. (1987, Cytometry 8, 315-320*),* Li,N., et al.: Mitochondrial complex I inhibitor rotenone induces apoptosis through enhancing mitochondrial reactive oxygen species production (2003, J Biol. Chem. 278, 8516-8525*)* or Verdoodt,B., et al.: Induction of polyploidy and apoptosis after exposure to high concentrations of the spindle poison nocodazole (1999, Mutagenesis 14, 513-520*).* Another disadvantage of this method is a relatively small accumulation of the synchronous fraction because a long-term exposure to microtubule poisons is not possible due to their high toxicity. The mitotic fraction therefore forms only a part of the total cell population and the collected cell fraction represents about 25-34%, as described in Zieve,G.W., et al: Production of large numbers of mitotic mammalian cells by use of the reversible -microtubule inhibitor nocodazole. Nocodazole accumulated mitotic cells (1980, Exp. Cell Res. 126, 397-405*).*

Separation methods use differences between the cells in particular phases of the cell cycle. These differences can be physical, when the size depends on the amount of DNA, biochemical, when the surface markers are examined, or physiological involving the examination of the cell adhesion to the cell culture substrate.

The easiest method of this kind is the separation by mitotic shake-off or wash-out, taking advantage of the fact that at a certain phase of the cell cycle, the mitotic cells are poorly adhered to the culture substrate, as described e.g. in Terasima,T. and Tolmach,L.J.: Growth and nucleic acid synthesis in synchronously dividing populations of HeLa cells (1963, Exp. Cell Res. 30, 344-362*).* Mitotic cells of most mammal cell lines can thus be separated using fundamental physical forces like washing out by a stream of fluid as described, e.g., in the document US 2002/0123144, or by shaking the culture plate using rolling (US 3871955) or vibrating (EP 1 284 285) motion. The advantage of this method is, that it is the only separation method that does not necessarily need any special equipment consisting of complicated and expensive instruments. This method primarily does not require usage of synchronization chemicals, even though the accumulation of mitotic cells is in that case very low, and therefore represents the least stressful method for the cells. The process of separation can be performed in an aseptic box, which decreases the contamination risk. The disadvantage is the aforementioned little accumulation of mitotic fraction, as the mitotic cells form only 1-3 % of a normal exponentially growing cell population and not all are successfully separated. To increase the number of accumulated mitotic cells, this method is often combined with a method using the mitotic inhibitors, i.e., using the microtubule poisons, which - as mentioned above - are highly toxic. The method can also be combined with storing every shaken-off or washed-out mitotic fraction on ice and after reaching a sufficient number of cells, the cultivation is transferred to normal culture conditions, as described in Fox,M.H.: Methods for Synchronizing Mammalian Cells (2004, In Cell Cycle Checkpoint Control Protocols*).* This allows for a possibility to avoid toxic mitotic inhibitors to gain higher amount of mitotic cells, but the technological intensity of the procedure increases significantly and physiological problems connected to the thermal shock may also arise.

Separation using counterflow centrifugal elutriation is based on different sizes of the cells. It is primarily determined by the size of the nucleus, which forms most of the cell's volume and its size reflects the phase of the cell cycle. It is possible to separate the cells according to their size in a special cell chamber using the combination of centrifugal force and force in the opposite direction produced by the flowing fluid. The procedure uses a special instrument, the so-called "Elutriator" described in Chang,Q., et al.: Counterflow centrifugal elutriation as a method of T cell depletion may cause loss of immature CD34+ cells (1997, Bone Marrow Transplant. 19, 1145-1150*)* or in Bachere,E., et al: Separation of Crassostrea gigas hemocytes by density gradient centrifugation and counterflow centrifugal elutriation (1988, Dev. Comp Immunol. 12, 549-559*).* The advantage of this method is that elutriation does not involve usage of any cell-coloring chemicals and provides for the separation of great number of cells in a relatively short time. The disadvantages are the high purchasing price of the elutriator and the fact that its maintenance and operation is quite time-consuming. During the elutriation, many cells are lost and processing a very high number of cells at a time is therefore necessary, which results in increased demands on their production. The acquired fractions are not completely homogeneous because the size of the cell does not precisely determine the phase of the cell cycle. During elutriation, the cells are exposed to non-physiological stress conditions, when adherent cell cultures must be transformed into suspension, the elutriation medium contains N,N,N',N'-ethylendiamintetraacetic acid (EDTA), the cells are exposed to fluctuation of temperature and additional stress factor can be also the amount of dissolved gases in the elutriation medium. During the procedure itself, risk of bacterial or yeast contamination of the cell culture is increased.

Another separation method is based on flow cytometry, interpreting the fluorescent signal from the individual cells on a special instrument - FACS, which can be supplemented by a sorting instrument, i.e., cell separator, as described in article Rieseberg,M., et al.: Flow cytometry in biotechnology (2001, Appl. Microbiol. Biotechnol. 56, 350-360*).*

Efficient FACS-Sorters separate cells based on their fluorescent signal strength. Fluorescent dyeing exists in a form of direct chemical dyeing, intermediated dyeing using antibodies with conjugated fluorescent marker or by artificially inserted genes coding the fluorescent proteins. The advantage of this method is that it is a relatively fast and precise method of cell population separation allowing more than one selection criterion. The disadvantage is the high purchase price, very complicated operation and maintenance of the instrument and also high prices of fluorescent dyes. For the synchronous population selection, fluorescent DNA dyeing is necessary, which can have mutagenic effect. During the sorting, cells are exposed to intensive laser light that can initiate production of toxic photoproducts. During the sorting, the cells are exposed to non-physiological stress conditions, when adherent cell cultures must be transferred into suspension culture before sorting, the sorting medium includes EDTA, and cells are exposed to fluctuation of temperature and non-physiological amount of dissolved gases. During the procedure itself, risk of bacterial or yeast contamination of the cell culture is increased.

The invention aims at eliminating the negative effects of the commonly used synchronization methods and at the same time decreasing the financial, time and operational intensity of this important laboratory procedure, while using the principle of anchorage dependence. It is defined as the proliferation's need of anchorage, i.e., adhesion, to the culture substrate and its description can be found e.g. in Assoian,R.K. and Zhu,X.: Cell anchorage and the cytoskeleton as partners in growth factor dependent cell cycle progression (1997, Curr. Opin. Cell Biol. 9, 93-98) nebo v Zhu,X., et al.: Adhesion-dependent cell cycle progression linked to the expression of cyclin D1, activation of cyclin E-cdk2, and phosphorylation of the retinoblastoma protein (1996, J. Cell Biol. 133, 391-403*).* Anchorage dependence concerns vast majority of adherently growing mammal cells and if the anchorage to the culture substrate is not possible or when it is interrupted, the proliferation is blocked. The invention uses and expands this principle.

### Disclosure of the Invention

The aforementioned aim is achieved by the provision of a method for production of a synchronized adherently growing cell line in late telophase, the subject matter of which consists in subjecting a culture bottle with culture medium containing adherently growing cell line to vibration under standard cultivation conditions an incubator without using any synchronizing chemicals, for a period of time from 4 to 30 hours. The vibration causes the washout of the poorly adhering mitotic cells to the culture medium and initiates inhibition of their proliferation. After finishing the vibrational treatment, the washed-out cell fraction suspension is removed and transferred to a new culture bottle or taken for analysis.

The term "synchronized adherently growing cell line" denotes cells of an adherent cell line, which are all in the same phase (or stage) of the cell cycle. The term "cell line" covers herein immortal cell lines, cell strains and primary cultures of adherent cells.

The standard cultivation conditions are well known to those skilled in the art. Commonly they are 37 °C, 5% CO₂, 100% humidity.

The novelty and inventiveness of the present invention consists in the fact that when culture surface is removed during mitosis in the metaphase stage, the unanchored adherently growing cell will proceed in mitosis only until karyokinesis, i.e. until nuclear segmentation, and the following cytokinesis, i.e. the final cell division into two daughter cells, will not occur. The cells then remain in the stage of incomplete cytokinesis or, in other words, in late telophase. Further proliferation of these cells is stopped until the cells are once again anchored on the culture surface. The present invention thus provides for induced synchronization of cells in late telophase which occurs as a result of the change of the culture conditions.

In a preferred embodiment, the vibration is defined by the frequency in the range of from 1 to 100 Hz (vibrations per second), more preferably in the range of from 1 to 50 Hz, most preferably in the range of from 5 to 40 Hz and by the amplitude in the range of from 0.001 to 30 mm, more preferably in the range of from 0.1 to 10 mm.

The culture bottle should be subjected to the vibrational treatment for a period of time that is sufficient for at least some cells to reach the metaphase stage, during which they are removed from the bottle inner surface by the action of vibrations. Preferably, the period of time of the vibrational treatment should be selected so that a significant or a desired proportion of the cells is removed from the bottle inner surface, but they do not develop stress for not being able to adhere to the bottle inner surface to continue their cell cycle.

The culture bottle is subjected to the vibrational treatment for a period of time of from 4 to 30 hours, more preferably in the range of from 4 to 24 hours, most preferably in the range of from 6 to 18 hours.

When performing the method of the present invention with a cell line with a hard-to-wash-out mitotic fraction, an inert abrasive, which makes the washing out of the mitotic cells easier, can be added into the medium before starting the vibrational treatment. The inert abrasive may be, e.g., sterilized agarose.

Another object of the invention is a device for carrying out the method of production of synchronized adherently growing cell lines seeded in culture bottles with culture medium. The device comprises a movable platform, which is flexibly mounted on a stationary support structure, provided with attachment means for attaching the culture bottle, and connected to a vibrator. The vibrator is connected by a cable to a vibration regulator embedded in a box. The box preferably holds an energy supply and is a part of the support structure or is firmly connected with the support structure. The energy supply is preferably a battery power supply.

Preferably, the platform can be flexibly mounted by using elastic brackets made of an elastomeric material, or spiral springs mounted on antipodal hinges.

It is also preferable to use a motor with unbalanced rotor as the vibrator.

In general, the attachment means can be any means suitable for firmly attaching the cultivation bottle to the movable platform, so that the cultivation bottle remains attached to the movable platform during the vibrational treatment. Preferably, the attachment means can be fixtures or fittings, particularly elastic strips.

The advantage of the present invention consists in the fact that it does not use any synchronization chemicals and does not change the chemical composition of the culture medium, but it allows for achieving large proportions of synchronized cells. Cells synchronized using this method do not show any measurable signs of stress and allow for a subsequent long-term cultivation. Easy applicability and automation of the method is also an important factor. The invention further includes an easily manufacturable device for carrying out the method of the invention, which provides for accumulation of a relatively big amount of chemically non-affected synchronized cells with minimum operational costs and zero demands for additional laboratory equipment. It is a compact module, which due to its size and technical realization can be placed into a standard cell incubator. The battery power supply enables a long operation inside the incubator under standard cultivation conditions without the need for intervention for a long enough period and the fitting of the device does not require any special technological modifications of the incubator or any change in the culture regime. The device uses mechanical forces acting on the cells significantly stronger in comparison with the so far used bacterial and suspension cells shakers, which means that both the wash-out of the uninhibited mitotic cells into suspension and initiation of inhibition of their proliferation occurs, and therefore the cells are arrested in late telophase.

### Brief description of the attached drawings

The present invention is further illustrated and described with reference to the attached drawings, wherein
Fig. 1 is a schematic view of a synchronization device with a standard cultivation bottle attached,
Fig. 2 is a schematic representation of one of the possible variants of a vibrator in the form of a contactless unbalanced motor,
Fig. 3 shows a comparison of population profiles (FACS profiles) of a normally growing cell population (chart A) and a population acquired by 18-hour cultivation shake-off using the instrument from Fig.1 (chart B) and

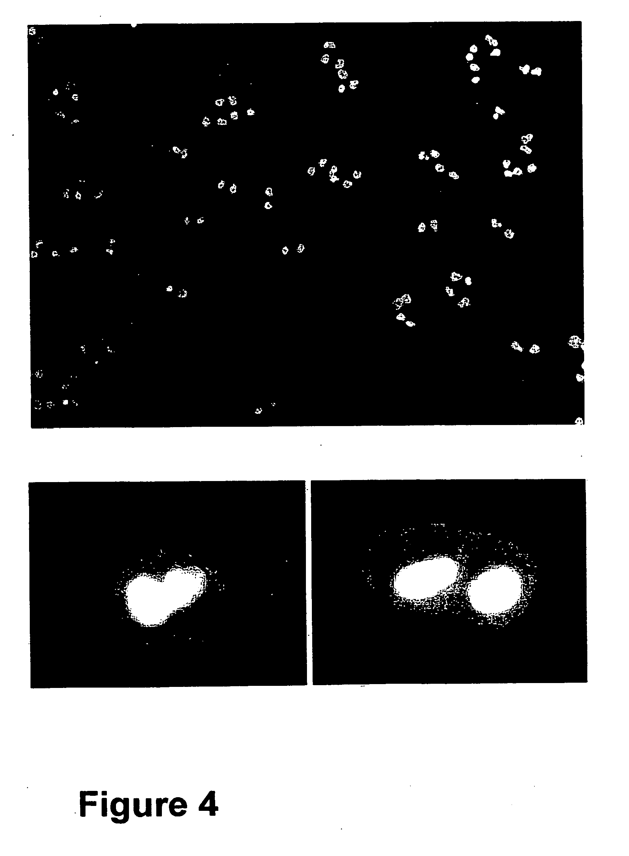

Fig. 4 shows the microscopic analysis of a cell population acquired by 24-hour cultivation using the instrument from Fig. 1, with most of the cells in late telophase, i.e. with 2 daughter nuclei,
Fig. 5 shows a photograph of a microscopic field depicting the visual analysis of different stages of mitosis in cells obtained by 6-hour cultivation shake-off using the instrument shown in Fig. 1 with emphasis on defective anaphases typical for cancer cell lines as represented by detail of anaphase cell with a bridge structure.

### Examples of carrying out of the Invention

### Example I

### Synchronization device

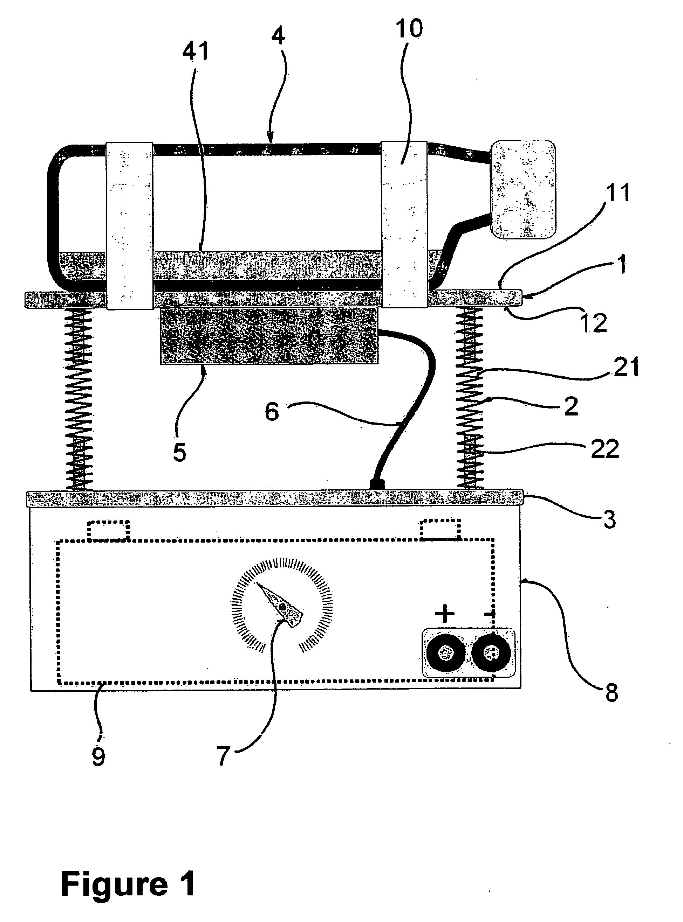

The synchronization device depicted in Fig. 1 consists of a movable platform 1, which is by means of elastic brackets 2 flexibly mounted on a stationary support structure 3. The elastic brackets 2 in this embodiment consist from spiral strings 21 mounted on antipodal hinges 22. The platform 1 is provided with attachment means 10, which are two elastic strips for securing the culture bottle 4 with culture medium 41 on the upper surface 11 of the platform 1. To the lower surface 12 of the platform 1, a vibrator 5 is attached, which is connected via a cable 6 to a vibration regulator 7 placed in a box 8, the upper side of which is formed by the support structure 3.

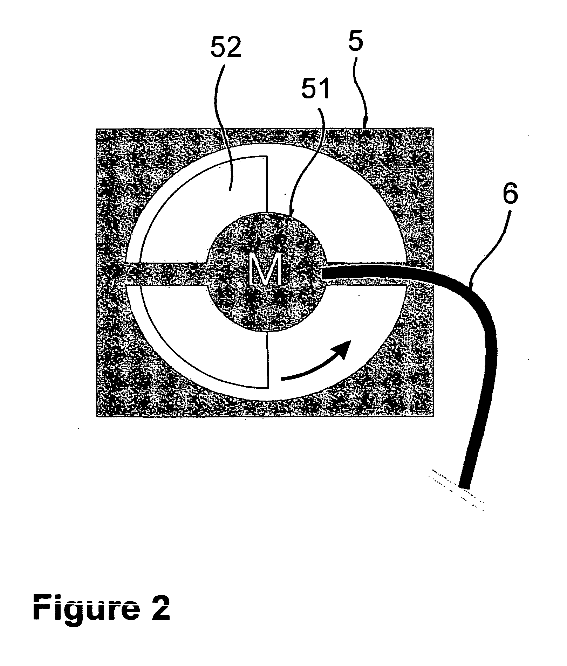

The vibrator 5, shown in more detail in Fig. 2, is electromechanical, consisting from a contactless motor 51 with an unbalanced rotor 52. The vibrator 5 is battery powered 9, for example by lead gel battery stored in the box 8 equipped with connectors for charging. The vibration regulator 7 is therefore actually a regulator engine speed governor 51.

When performing the synchronization of cell lines whose mitotic fraction is easily washed-out, the culture bottle 4 with culture medium 41 containing exponentially growing adherent cell line is attached to the movable platform 1 of the instrument using the attachment means 10 and the instrument is placed into an incubator. Subsequently, it is turned on, and adequate mechanical forces acting on cells are set by setting a suitable vibration frequency and amplitude and the cells are being incubated under standard cultivation conditions and constant vibrations for a pre-determined period of time. After the pre-determined period of time, the washed-out cell fraction is removed and transferred to a new culture bottle or a plate or directly processed for analysis. When performing the synchronization of cell lines with a hard-to-wash-out mitotic fraction, an inert abrasive, which makes the washing out of the mitotic cells easier, can be added into the medium before starting the vibrational treatment.

Specific experiment results of the aforementioned method of production of synchronized adherently growing cell lines are demonstrated by the following examples.

### Example 2

### Synchronization of U-2-OS human cell line

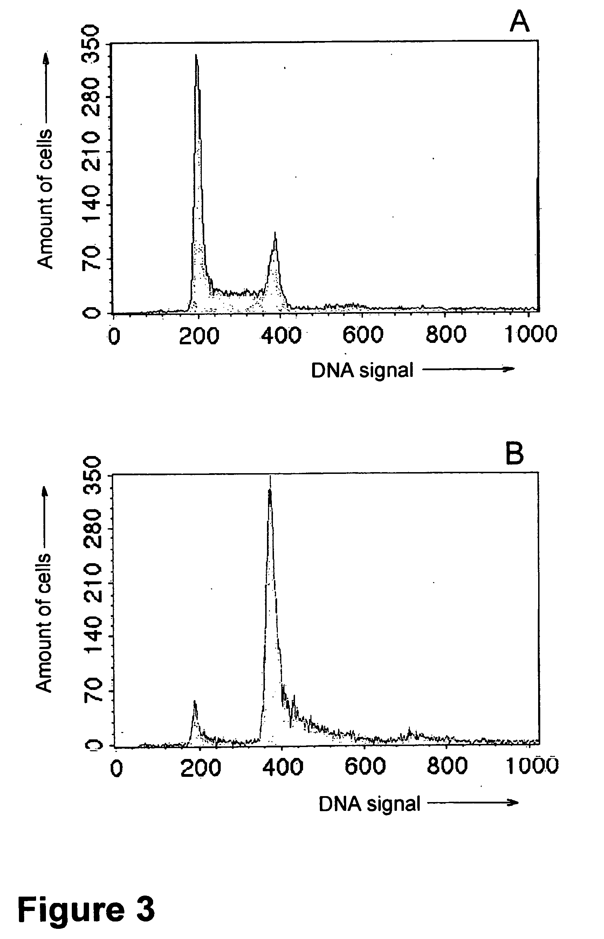

Adherently growing human cell line U-2-OS (osteosarcoma) of 9x10⁵ cells was placed in a standard culture bottle T75 (cultivation surface area 75 cm²) and cultivated in standard culture medium consisting of DMEM 90 % and fetal bovine serum 10 % under standard cultivation conditions i.e., humidity 100 %, temperature 37 °C, CO₂ 5 % for 24 hours. Subsequently, the medium was replaced by a new one and the culture bottle was attached to the movable platform of the device described in Example 1, and the device with the bottle was placed into a standard Heraeus BB16 culture incubator under a standard cultivation regime, i.e., humidity 100 %, temperature 37 °C, CO₂ 5 %. After the placement into the incubator, the vibration mode of the device was manually activated and set to 30 vibrations per second and maximum amplitude 10 mm. After 18-hour incubation, the medium with the washed-out cells was removed. A part of the cell suspension was concentrated by 10-minute centrifugation at 500 g and immediately fixed by ethanol 70 %, stained for DNA content by propidium iodide and subsequently processed for FACS analysis, the result of which is shown in Fig. 3, chart B. The remaining part of the cell suspension was divided into four new cultivation Petri dishes and was left to incubate under standard cultivation conditions. The individual dishes were then removed after 4, 6, 9 and 15 hours and processed for FACS analysis to monitor the cell cycle progress.

### Example 3

### Analysis of anaphase bridges in anaphase U-2-OS human cells obtained by 6-hour incubation on the device

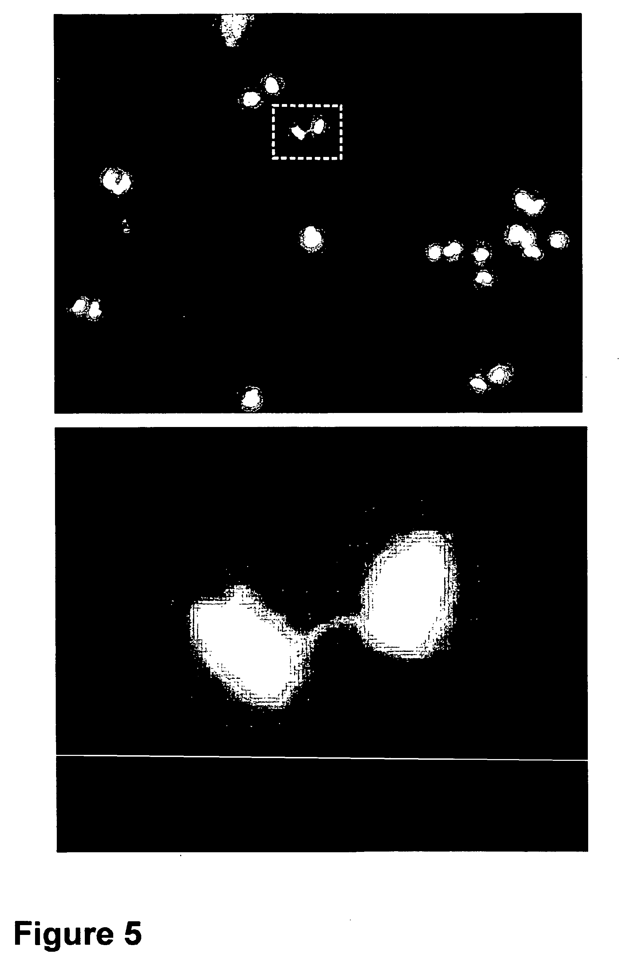

Shorter incubation times within 1-8 hours on the devices yield less synchronized cells washed into the suspension but generally lower the stress and are leading to higher content of cells in earlier stages of mitosis than described in example 2, employing 18-hour cultivation, where most of the cells in the suspension are represented by the telophase cells. Thus, shorter incubation times on the device can be particularly useful for qualitative and quantitative analysis of different stages of mitosis. In this experiment we aimed on analysis of so-called anaphase bridges, defective structures typical for cancer cell lines.
Adherently growing human cell line U-2-OS (osteosarcoma) of 2x10⁶ cells was placed in a standard culture bottle T150 (cultivation surface area 150cm²) and cultivated in standard culture medium consisting of DMEM 90 % and fetal bovine serum 10 % under standard cultivation conditions i.e., humidity 100 %, temperature 37 °C, CO₂ 5 % for 24 hours. Subsequently, the medium was replaced by a new one and the culture bottle was attached to the movable platform of the device described in Example 1, and the device with the bottle was placed into a standard Heraeus BB16 culture incubator under a standard cultivation regime, i.e., humidity 100 %, temperature 37 °C, CO₂ 5 %. After the placement into the incubator, the vibration mode of the device was manually activated and set to 30 vibrations per second and maximum amplitude 10 mm. After 6-hour incubation, the medium with the washed-out cells was removed. The cell suspension was concentrated by 10-minute centrifugation at 500g and immediately fixed by ethanol 70 %, stained for DNA content by propidium iodide. Cells were fixed on a microscopic slide and examined using fluorescent microscope for visual assessment of cells in different stages of mitosis (prophase, metaphase, anaphase, telophase) representing the majority of the obtained cell population. Photograph of a microscopic field depicting the analysis and a detail of a cell with defective anaphase represented by anaphase bridge structure is shown in Fig. 5.

### Example 4

### Using synchronized U-2-OS line for analyzing expression of aphidicolin inducible common fragile sites

As a practical application of the method we used the synchronized population of U-2-OS cells to ensure that all cells in the population will be exposed during the whole S-phase (phase of DNA replication) to a replication stress inducer - aphidicolin.
To obtain synchronized cells adherent human cell line U-2-OS (osteosarcoma) of 9x10⁵ cells was placed in a standard culture bottle T75 (cultivation surface area 75cm²) and cultivated in a standard culture medium consisting of DMEM 90 % and fetal bovine serum 10 % under standard culture conditions for 24 hours. Subsequently, the medium was replaced by a new one and the culture bottle was attached to the movable platform of the device described in Example 1 and the device with the bottle was placed into a standard Heraeus BB16 culture incubator under standard cultivation regime, i.e. humidity 100%, temperature 37 °C, CO₂ 5%. After placing the bottle in the incubator, the vibration mode of the device was manually activated and set to 30 vibrations per second and maximum amplitude 10mm. After 18-hour incubation, the medium with the washed-out cells was removed. The cell suspension was divided into four new cultivation Petri dishes. One of the dishes carried out the control function and to the three other Petri dishes final concentration 0.1, 0.2 and 0.4 µM of Aphidicolin was added. The dishes were then cultivated under standard cultivation conditions. After 18 hours, the dishes were regularly controlled every hour under inversed microscope and the total number of rounded, i.e. mitotic, cells was recorded. When the rate of the mitotic cells in a certain dish reached approximately ten per cent, the dish was taken out to be examined for mitotic chromosome breakage by a standard procedure described for example in article Margaret A. Leversha, (1998, In Cell Biology: A Laboratory Handbook, Julio E.Celis, Ed. (ACADEMIC PRESS, San Diego), chapter 11, pp. 428-436*).*

### Example 5

### Synchronization of BJ human cell line

Adherently growing human BJ cell line (foreskin fibroblasts) of 10x10⁵ cells was placed in a standard culture bottle T75 (cultivation surface area 75cm²) and cultivated in standard culture medium consisting of DMEM 90% and fetal bovine serum 10% under standard culture conditions for 24 hours. Subsequently, the medium was replaced by a new one, 200 mg of sterilized agarose was added to fulfill a function of inert abrasive and make the wash-out of mitotic cells easier. Afterwards, the culture bottle was attached to the movable platform of the device as described in Example 1, and the device with the bottle was placed into a standard Heraeus BB16 culture incubator under standard cultivation regime, i.e., humidity 100%, temperature 37 °C, CO₂ 5%. After placing the bottle in the incubator, the vibration mode of the device was manually activated and set to 30 vibrations per second and maximum amplitude 10 mm. After 18-hour incubation, the medium with the washed-out cells and agarose was removed. The cell suspension was divided into five new cultivation Petri dishes and left to settle during 3-hour incubation under standard cultivation conditions. Subsequently, medium containing agarose was drained off, the cells were washed by phosphate buffer (PBS) and new standard culture medium was added. Further incubation followed, when the individual dishes were then removed after 1, 3, 6, 12 and 18 hours after the agarose wash-off and processed for FACS analysis to monitor the cell cycle progress.

### Industrial Applicability

Method of production of synchronized adherently growing cell lines and the device for carrying out this method is utilizable especially in biotechnological laboratories for basic and applied research engaged in *in-vitro* cultivations of adherent cell lines, which need to be for various reasons synchronized.

## Claims

1. A method for production of a synchronized adherently growing cell line in late telophase, **characterized in that** a culture bottle with culture medium containing adherent cell line is subjected to a vibration under standard cultivation conditions in an incubator, without using any synchronization chemicals, for a period of time of from 4 to 30 hours, and after finishing the vibrational treatment, the washed-out cell fraction suspension is removed and transferred to a new culture bottle or taken for analysis.

2. The method according to claim 1, wherein the vibration is defined by the frequency in the range of from 1 to 100 Hz and the amplitude in the range of from 0.001 to 30 mm.

3. The method according to claim 1, wherein the period of time is from 6 to 30 hours.

4. The method according to claim 1, wherein an inert abrasive is added into the culture bottle before starting the vibrational treatment.

5. A device for carrying out the method of production of synchronized adherently growing cell lines seeded in culture bottles (4) with culture medium (41), **characterized in that** it comprises a movable platform (1), flexibly mounted on a stationary support structure (3), said movable platform (1) being provided with attachment means (10) for attaching the culture bottle (4) and connected to a vibrator (5), said vibrator being connected by a cable (6) to a vibration regulator (7) embedded in a box (8), said box (8) holding an energy supply (9), wherein a motor (51) equipped with an unbalanced rotor (52) is provided as the vibrator (5).

6. The device according to claim 5, **characterized in that** the movable platform (1) is flexibly mounted by using elastic brackets (2).

7. The device according to claim 5, **characterized in that** the movable platform (1) is flexibly mounted by using spiral springs (21) freely mounted on antipodal hinges (22).

8. The device according to any of claims 5 to 7, **characterized in that** elastic strips are provided as the attachment means (10).

## Patentansprüche

1. Verfahren zur Erzeugung einer synchronisierten adhärent wachsenden Zelllinie in der späten Telophase, **dadurch gekennzeichnet, dass** eine Kulturflasche mit einem Kulturmedium, das eine adhärente Zelllinie enthält, einer Vibration unter Standardkultivierungsbedingungen ohne Verwendung von Synchronisationschemikalien in einem Inkubator für einen Zeitraum von 4 bis 30 Stunden ausgesetzt wird, und nach Beendigung der Vibrationsbehandlung wird die Suspension der ausgewaschenen Zellfraktion zu einer neuen Kulturflasche übertragen oder analysiert.

2. Verfahren nach Anspruch 1, wobei die Vibration durch eine Frequenz im Bereich von 1 bis 100 Hz und die Amplitude im Bereich von 0,001 bis 30 mm definiert ist.

3. Verfahren nach Anspruch 1, wobei die Zeitspanne von 6 bis 30 Stunden beträgt.

4. Verfahren nach Anspruch 1, wobei ein inertes Schleifmittel in die Kulturflasche vor Beginn der Vibrationsbehandlung zugegeben ist.

5. Vorrichtung zur Durchführung des Verfahrens zur Erzeugung einer synchronisierten adhärent wachsenden Zelllinie in Kulturflaschen (4) mit Kulturmedium (41) ausgesät, **dadurch gekennzeichnet, dass** sie eine auf einer ortsfesten Trägerstruktur elastisch montierte (3) bewegliche Plattform (1) hat, wobei die gesagte bewegliche Plattform (1) mit Befestigungsmitteln (10) zum Anbringen der Kulturflasche (4) vorgesehen ist und an einem Vibrator (5) angeschlossen ist, wobei der gesagte Vibrator durch ein Kabel (6) mit einem Vibrationsregulator (7) verbunden ist, welcher in einem Kasten (8) eingebettet ist, wobei der Kasten (8) eine Energieversorgung (9) besitzt, wobei ein Motor (51) mit einem Unwuchtrotor (52) als der Vibrator (5) vorgesehen ist.

6. Vorrichtung nach Anspruch 5, **dadurch gekennzeichnet, dass** die bewegliche Plattform (1) unter Verwendung von elastischen Konsolen (2) flexibel montiert ist.

7. Vorrichtung nach Anspruch 5, **dadurch gekennzeichnet, dass** die bewegliche Plattform (1) unter Verwendung von frei montierten Spiralfedern (21) auf antipodalen Scharnieren (22) flexibel montiert ist.

8. Vorrichtung gemäß einem der Ansprüche 5 bis 7, **dadurch gekennzeichnet, dass** elastische Bänder als Befestigungsmitteln (10) vorgesehen sind.

## Revendications

1. La méthode de la production des lignes des cellules adhérentes synchronisées en télophase tardive, **caractérisée en ce qu'**une bouteille de culture avec le médium de culture contenant une ligne adhérente de cellules est soumise aux vibrations dans l'incubateur lors des conditions standard de culture, sans utilisation des produits chimiques de synchronisation, pendant une durée de 4 jusqu'à 30 heures, et après la fin des vibrations, on enlève la suspension de la fraction non-adhérente des cellules et on la transfère dans une nouvelle bouteille de culture ou on la prélève pour l'analyse.

2. La méthode selon la revendication 1 où la vibration est définie par la fréquence de 1 à 100 Hz et l'amplitude de 0,001 à 30 mm.

3. La méthode selon la revendication 1 où la durée est de 6 à 30 heures.

4. La méthode selon la revendication 1 où il est ajouté de l'abrasif inerte à la bouteille de culture avant le début des vibrations.

5. L'appareil pour effectuer la méthode de la production des lignes des cellules adhérentes synchronisées implantées dans les bouteilles de culture (4) avec le médium de culture (41) **caractérisé en ce qu'**il contient une plate-forme mobile (1) qui est librement accrochée à une construction de base fixe (3) et la plate-forme mobile (1) est pourvue des dispositifs de fixation (10), pour la fixation de la bouteille de culture, (4) et elle est jointe au vibrateur (5) et le vibrateur mentionné est joint par un câble (6) avec un régulateur (7) du vibrateur placé dans une boîte (8), et la boîte mentionnée (8) porte une admission (9) de l'énergie où le vibrateur (5) est un moteur (51) équipé d'un rotor non-équilibré (52).

6. L'appareil selon la revendication 5 **caractérisé en ce que** la plate-forme mobile (1) est librement accrochée à l'aide des supports flexibles (2).

7. L'appareil selon la revendication 5 **caractérisé en ce que** la plate-forme mobile (1) est librement accrochée à l'aide des ressorts enroulés (21) librement mis sur les axes opposés (22).

8. L'appareil selon une quelconque revendication de 5 à 7 **caractérisé en ce qu'**en tant que dispositifs de fixation (10) sont offerts des suspentes élastiques.
